# EUROPEAN PATENT APPLICATION

(11) **EP 1 106 182 A1**
(43) Date of publication of application: **13.06.2001**
(21) Application number: 99937009.1
(22) Date of filing: 10.08.1999
(51) Int. Cl.: A61K 38/18, A61K 45/00

(54) **BLOOD SUGAR LEVEL CONTROLLING AGENT**

(30) Priority: 11.08.1998 JP 22644298
(71) Applicant: Sumitomo Pharmaceuticals Company, Limited, Osaka 541-8510 (JP)
(72) Inventor: NAKAGAWA, Tsutomu, Toyonaka-shi, Osaka 561-0802 (JP); TAIJI, Mutsuo, Takatsuki-shi, Osaka 569-1044 (JP); NAKAYAMA, Chikao, Sanda-shi, Hyogo 669-1323 (JP); NOGUCHI, Hiroshi, Kawanishi-shi, Hyogo 666-0143 (JP)
(74) Representative: VOSSIUS & PARTNER
(86) International application number: JP9904322
(87) International publication number: WO0009147

(57) **Abstract**

A blood glucose level controlling agent for a diabetic patient to be insulinized, which comprises as the active ingredient an insulin receptor agonist and a neurotrophin; a use of these two ingredients in preparation of said blood glucose level controlling agent; and a method for controlling the blood glucose level of a diabetic patient within the normal range by using these two ingredients. By administering the pharmaceutical composition of the present invention for controlling the blood glucose lever of a patient to be insulinized, the effect of insulin is enhanced, while side effects thereof such as hypoglycemic shock are eased, thereby also enabling improvement in the compliance.

## Description

### TECHNICAL FIELD

The present invention relates to a medicament controlling or normalizing the blood glucose level of diabetic patients to be insulinized within the normal range. More particularly, the present invention relates to a blood glucose level controlling agent for a diabetic patient to be insulinized, which comprises as the active ingredient an insulin receptor agonist and a neurotrophin, a use of these two ingredients in preparation of said blood glucose level controlling agent, and a method for controlling the blood glucose level of patients to be insulinized within the normal range by using said two ingredients.

### BACKGROUND ART

Diabetes is classified into type 1 and type 2 based on the pathogenesis thereof. Type 1 diabetes mellitus is caused by the lack of the insulin secretion due to the destruction of pancreatic beta cells, which is induced by virus infection or autoimmune response, etc. In general, juvenile-on set diabetes is classified in this category, wherein ketosis highly appears so that there is a possibility of danger in life. In addition, the continuance of chronic hyperglycemia may cause diabetic complications due to microangiopathy, for example, diabetic retinopathy, diabetic nephropathy, diabetic neuropathy, etc.

Recently, the number of patients afflicted with type 2 diabetes mellitus has been increased, due to an improvement in a living standard, and/or change of the dietary life into the Western style, or increase in lack of exercise. Various diabetic complications may be induced by continuance of hyperglycemia in type 2 diabetes mellitus, which is the same as in type 1 diabetes mellitus.

In the current clinical treatment of diabetic patients, insulin injections is mainly employed in treatment of patients with type 1 diabetes mellitus. Recently, according to the report that a strict control of blood glucose level is effective in the prophylaxis of the onset of diabetic complications, the frequent injection of insulin, i.e., the insulin consolidation therapy has positively been employed (cf. Nippon Rinsho, vol. 55, 1997 suppl., p. 249-254). However, the insulin therapy also has problems, and the frequent injection of insulin gives a large burden to the patients. Moreover, hypoglycemia is also a serious problem, which is induced by overaction of insulin (cf. Nippon Rinsho, vol. 55, 1997 suppl., p. 243-248). Further, there is a report that patients to be treated with the insulin consolidation therapy showed a higher rate of risk of onset of macrovascular diseases such as myocardial infarction (cf. N. Eng. J. of Med., vol. 329, p. 977 (1993)).

Type 2 diabetes mellitus is basically treated by exercise therapy or dietary therapy, and if necessary, it is treated by administration of an oral antidiabetic agent such as an SU agent, a biguanide compound, etc. However, in spite of these therapies, there are many cases wherein the blood glucose level cannot sufficiently be controlled in the patients with progressed diabetes mellitus, and hence in those patients, insulin is administered (cf. Nippon Rinsho, vol. 55, 1997 suppln., p. 233-237). Besides, recently, in patients afflicted with type 2 diabetic mellitus, it is increased to positively treat with insulin even at the early stage of the disease. Thus, the efficacy of insulin treatment to patients has been realized again. However, there are still remained problems in the insulin treatment of type 2 diabetic mellitus as well as in the treatment of type 1 diabetes mellitus, such as decrease in the compliance, onset of hypoglycemia, and development of macrovascular diseases.

In order to compensate for the above defects of insulin therapy, there has been studied on a combined use of insulin and another pharmacologically active substance exhibiting the hypoglycemic activity which is contrary to insulin, such as somatostatin (US 4,703,034), amylin agonist compounds, AC137 (US 5,175,145), etc.

Neurotrophic factors are proteins, which are provided from target cells or neurons and glia cells·Schwann cells in the living body, and show activities to maintain the survival and differentiation of neurons, and are classified into many types according to the kinds of nerves or receptors to function. Among them, proteins being known as neurotrophins have high structural homology with each other. The typical examples thereof are an NGF (nerve growth factor), a BDNF (brain-derived neurotrophic factor), an NT-3 (neurotrophin 3), an NT-4/5, etc., and they are known to act as a specific ligand of receptors (trkA, trkB and/or trkC), which are the products of P-75 and trk genes (cf. Takeshi NONOMURA, Hiroshi HATANAKA; Jikken Igaku, vol. 13, p. 376 (1995)).

Neurotrophins such as NGF, BDNF, etc. have been studied as a therapeutic agent for treating a patient of neurodegenerative diseases such as ALS. For example, Journal of Neuropathology and Experimental Neurology, vol. 56, p. 1290 (1997) A.P. Mizisin et al. discloses the pharmacological activity of BDNF on diabetic peripheral neuropathy, but this literature merely suggests the possible pharmacological activity of BDNF on diabetic peripheral neuropathy based on the finding that BDNF improves the reduction of motor nerve conduction *in vivo.*

In the following exemplified reports, the effects of neurotrophic factors on the blood glucose level are reported.
(1) Exp. Neurology, vol. 131, p. 229 (1995), Palley-mounter MA et al.:
   The appetite suppression induced by BDNF is studied on normal mice. It discloses that by administration of BDNF, the blood insulin level is reduced, and the appetite and the body weight thereof are also reduced, but the blood glucose level is not reduced.
(2) Cytokine, vol. 7, p. 150 (1995), Fantuzzi, G. et al.:
   The effects of CNTF (ciliary neurotrophic factor) on normal mice are reported. It discloses that by administration of CNTF, the appetite and the body weight of the mice are reduced, and the blood glucose level thereof is also reduced. The object of this literature is the clarification of the mechanism of inflammation inducing activity of CNTF, or other cytokine members such as IL-1, IL-6, but this literature never discloses the relation between those and diabetes mellitus.
(3) Proc. Natl. Acad. Sci. USA, vol. 94, p. 6456 (1997), Gloaguen, I. et al. and WO 98/22128, Istitute di Ricerche di Biologia Molecolare P. Angeletti S.P.A. (published on May 28, 1998) :
   By administration of CNTF to db/db mice, obese diabetic animal models, the reduction of the body weight and the appetite caused thereby, and the blood glucose level reducing effect of CNTF are reported. WO 98/22128 discloses a method for treatment of obesity or obese diabetic mellitus, comprising administration of CNTF receptor activator. However, these literatures never disclose or suggest the effects of CNTF on type 1 diabetes mellitus, nor a combined use of CNTF and insulin.
(4) Biochem. Biophys. Res. Commun., vol. 238, p. 633 (1997), One M. et al.:
   It reports that by administration of BDNF or NT-3 to db/db mice that are obese diabetic animal models, the hyperglycemia can be suppressed, being not simply caused by the reduced food uptake, and the blood glucose level can be reduced to the normal mice level. In addition, it also reports that the administration of BDNF alone did not show effects on blood glucose level in streptozotocin-induced diabetic model rats, the pancreatic-cells of which were damaged by streptozotocin and could not secrete insulin.

In the clinical field, a medicament which can assure and help the blood glucose level control by insulin with more safety and more accuracy, which is important in the treatment of type 1 diabetes mellitus and type 2 diabetes mellitus, and a hypoglycemic agent which can enable improvement in the compliance and ease side effects such as hypoglycemic shock, macrovascular diseases, etc. have been desired.

### DISCLOSURE OF INVENTION

An object of the present invention is to provide a novel agent for controlling blood glucose level, which can enhance the effects of insulin, and reduce the risk of side effects thereof such as hypoglycemic shock and further can enable improvement in the compliance.

Another object of the present invention is to provide a medicament controlling the blood glucose level of diabetic patients to be insulinized, which comprises as the active ingredient an insulin receptor agonist and a neurotrophin.

Further object of the present invention is to provide a use of said two ingredients in preparation of said agent for controlling blood glucose level.

Still further object of the present invention is to provide a method for controlling the blood glucose level of patient to be insulinized within the normal range by simultaneously or separately administering an insulin receptor agonist and a neurotrophin while the onset of the side effects is suppressed.

The present inventors have studied on the effects of BDNF, which exhibits a distinguished blood glucose level reducing activity in animal models for type 2 diabetic mellitus by administration of BDNF alone, but does not reduce the blood glucose level to a level below the normal range, and further studied on a combined use of BDNF and insulin in diabetic animal models of which the secretion of insulin is reduced. As a result, we have found (1) that by administration of BDNF together with insulin of an amount at which insulin cannot exhibit sufficient effects when administered alone, BDNF amplifies the effects of insulin and reduces the blood glucose level of diabetic animal models of which the secretion of insulin is reduced into a preferable value, and (2) that such effects of BDNF are not additive/synergic effects, and when BDNF is administered together with insulin of a much higher amount, the above effect of BDNF becomes smaller and BDNF does not reduce the blood glucose level into a less than normal value, and finally, the present invention has been accomplished.

Namely, the present invention has the following features.
[1] A blood glucose level controlling agent for a diabetic patient to be insulinized, which comprises as the active ingredient an insulin receptor agonist and a neurotrophin.
[2] The blood glucose level controlling agent according to [1], wherein the neurotrophin is a brain-derived neurotrophic factor (BDNF).
[3] The blood glucose level controlling agent according to [1], wherein the insulin receptor agonist is an insulin.
[4] The blood glucose level controlling agent according to [1], wherein the diabetic patient to be insulinized is a patient afflicted with insulin dependent diabetes mellitus.
[5] A blood glucose level controlling agent for a diabetic patient to be insulinized, which comprises as the active ingredient an insulin receptor agonist, and a trkA, a trkB and/or a trkC receptor agonist.
[6] An auxiliary agent for insulin therapy, which comprises as the active ingredient a neurotrophin.
[7] A use of an insulin receptor agonist and a neurotrophin in preparation of a blood glucose level controlling agent for a diabetic patient to be insulinized.
[8] A method for controlling the blood glucose level of a diabetic patient to be insulinized within the normal range, which comprises simultaneously or separately administering to said diabetic patient an insulin receptor agonist and a neurotrophin.

### BRIEF DESCRIPTION OF DRAWINGS

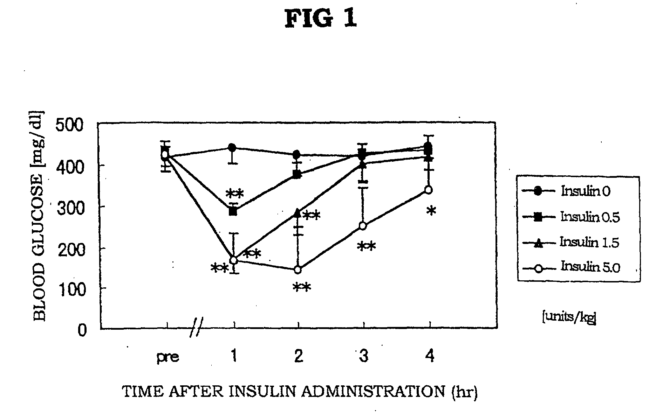

Fig. 1 is a graph indicating the relation between the insulin dose and the blood glucose reducing activity in STZ-induced diabetic mice. The axis of ordinate indicates the blood glucose level and the axis of abscissa indicates the time after insulin administration. ● indicates the average blood glucose levels at each point in the group treated with insulin 0 unit/kg (n=6), ■ indicates those in the group treated with insulin 0.5 unit/kg (n=6), ▲ indicates those in the group treated with insulin 1.5 unit/kg (n=6), and ○ indicates those in the group treated with insulin 5.0 units/kg (n=6). * and ** indicate that there is a significant difference of p<0.05, P<0.01, respectively, as compared with the group treated with insulin 0 unit/kg, in Turkey's test.

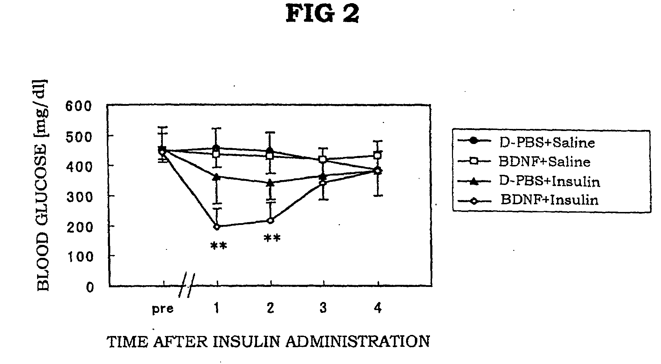

Fig. 2 is a graph indicating the effects of a combined use of BDNF and insulin of a low dose in STZ-induced diabetic mice. The axis of ordinate indicates the blood glucose level and the axis of abscissa indicates the time after insulin administration. ● indicates the average blood glucose levels at each point in the group treated with D-PBS + saline (n=10), □ indicates those in the group treated with BDNF + saline (n=10), ▲ indicates those in the group treated with D-PBS + insulin (n=10), and ◇ indicates those in the group treated with BDNF + insulin (n=9). ** indicates that there is a significant difference of P<0.01 as compared with the group treated with D-PBS + insulin in Turkey's test.

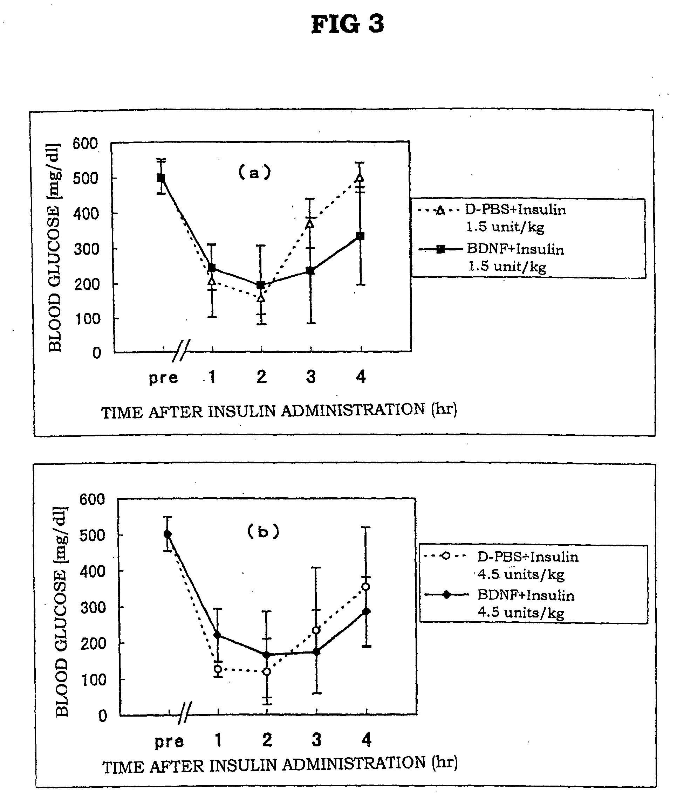

Fig. 3 is a graph indicating the effects of a combined use of BDNF and insulin of a high dose in STZ-induced diabetic mice. The axis of ordinate indicates the blood glucose level and the axis of abscissa indicates the time after insulin administration. (a): ◇ indicates the average blood glucose levels at each point in the group treated with D-PBS + insulin 0.5 unit/kg (n=6), and ■ indicates those in the group treated with BDNF + insulin 1.5 unit/kg (n=6). (b): ○ indicates the average blood glucose levels at each point in the group treated with D-PBS + insulin 4.5 units/kg (n=6), and ◆ indicates those in the group treated with BDNF + insulin 4.5 units/kg (n=6). There was no significant difference in the blood glucose levels between the D-PBS-treated groups and the BDNF-treated groups at each dose of insulin.

### BEST MODE FOR CARRYING OUT THE INVENTION

The meaning or definition of each term used in the present specification is explained below.

The "diabetic patient to be insulinized" means a patient who needs insulin treatment due to the lack of insulin activity. More particularly, the "diabetic patient to be insulinized" means a patient afflicted with type 1 diabetic mellitus or type 2 diabetic mellitus being in the insulin-dependent condition, who needs insulin in order to treat the hyperglycemia. For example, it means a patient who shows hyperglycemia because insulin is not secreted normally by reason that pancreatic β cells are damaged.

The "insulin receptor agonist" is a generic name for substances which can be bound to an insulin receptor, and can activate said receptor, and consequently, can reduce the blood glucose level. Concretely, it includes, for example, naturally occurred human insulin, a modified insulin such as rapidly-acting modified insulin LY275585, IGF-1, etc. A variant of insulin or modified IGF-1 is a protein having a primary structure wherein some amino acid residues of the naturally-occurred amino acid sequence are modified by a substitution, a deletion or an addition by conventional genetic engineering techniques, and it is also included within the scope of the "insulin receptor agonist" of the present invention as far as such product shows an insulin receptor binding ability and an activating activity of insulin receptor. In addition, a peptide of a lower molecular weight or an organic compound may be included within the scope of the "insulin receptor agonist" of the present invention as far as such product shows an insulin receptor binding ability and an activating activity of insulin receptor.

The "neurotrophin" means a neurotrophic factor, which is secreted from the target cells for nerve growth, or promotes the growth, differentiation, or survival of neurons by autocrine or paracrine, and makes them form a neural circuit (synapse) in the living body. For example, a brain-derived neurotrophic factor (hereinafter, abbreviated as BDNF), a nerve growth factor (hereinafter, abbreviated as NGF), a neurotrophin 3 (hereinafter, abbreviated as NT-3), a neurotrophin 4 (hereinafter, abbreviated as NT-4), a neurotrophin 5 (hereinafter, abbreviated as NT-5), and a neurotrophin 6 (hereinafter, abbreviated as NT-6) are known at present.

The "blood glucose level controlling agent for a patient to be insulinized" means a medicament that is used for controlling the blood glucose level of the patient who is suffered from type-1 or type 2 diabetic mellitus and can be a subject of insulin treatment within the normal range. The normal blood glucose level is, according to the present criterion of clinical examinations, is less than 200 mg/dl two hours after the tolerance test of glucose at the dose of 75 g, and less than 140 mg/dl of fasting blood glucose, especially in the range of 100 to 120 mg/dl. The blood glucose level controlling agent of the present invention is a medicament that enables the safe and continuous control of blood glucose level within the normal range, which can reduce the blood glucose level of a patient whose fasting blood glucose level is more than 400 mg/dl into 200 mg/dl or less, but not less than 70 mg/dl.

The blood glucose level of diabetes is, according to the recently revised criterion of diagnosis for diabetes, is more than 200 mg/dl two hours after the tolerance test of glucose at the dose of 75 g, and more than 126 mg/dl of fasting blood glucose.

The "trkA, trkB and/or trkC receptor agonist" is a generic name of substances that are bound to trkA, trkB or trkC and activate them to exhibit their activities, among the trk gene expression products being known as receptors for "neurotrophin". Concretely, the known neurotrophin is, for example, NGF binding to trkA, BDNF or NT-4 binding to trkB, and NT-3 binding to trkC, etc. This concept includes not only a modified compound (modified by amino acid substitution, deletion or addition), but also peptides of a lower molecular weight and organic compounds as far as they exhibit a binding ability and activating ability to trkA, trkB or trkC receptor, for example, phosphorization activity of tyrosine residue.

The "auxiliary agent for insulin treatment" means a medicament which exhibits (1) an activity of reducing or lowering the dose of insulin and the administration frequency of insulin, and (2) an activity of prevention of serious hypoglycemia by overaction of insulin, by administering prior to or simultaneously insulin administration in a patient with type 1 or type 2 diabetes mellitus under insulin treatment. The effect of (1) is the same meaning as "an activity to enhance insulin action". This effect may be achieved by increasing insulin sensitivity of the cells and it is especially appeared when administered together with insulin of a low dose at which insulin cannot exhibit a sufficient hypoglycemic activity when administered alone. The effect of (2) is appeared when administered together with insulin of a high dose at which insulin can exhibit sufficient hypoglycemic activity even when administered alone.

### METHOD FOR PREPARATION AND ASSAY

Neurotrophin (an NGF, a BDNF, an NT-3, an NT-4, etc.) to be used in the present blood glucose level controlling agent or in the present auxiliary agent for insulin treatment for a patient to be insulinized may be any one, for example, a naturally-occurred one, a recombinant one, as far as it shows its inherent biological activities. It can be used in the present invention with purification. For example, BDNF was isolated from porcine brain by Barde, Y.E. et al (cf. The EMBO Journal, vol. 5, p. 549-553 (1982)), and BDNF gene was analyzed after cloning, whereby it was confirmed that BDNF has a primary structure consisting of 119 amino acids (cf., Leibrock, J. et al., Nature, vol. 341, p. 149 (1989)). In addition, a modified recombinant BDNF such as Met-BDNF having a methionine residue at the N-terminus, or a recombinant mutant BDNF produced by a substitution, a deletion or a removal, or an addition of a part of amino acid sequence of the above naturally occurred BDNF sequence by a conventional gene engineering technique may be used in the present invention, as far as they are a pharmaceutical preparation having a BDNF activity.

The "BDNF activity" means activities of maintaining survival or promoting differentiation of a dorsal root ganglia, a ganglion inferius nervi vagi, a motoneuron, a retina ganglia, a nigradopaminergic neuron, a basal forebrain cholinergic neuron, etc. These activities can be confirmed in vitro or in vivo (JP-A-5-328974, USP 5,180,820).

Various methods for preparing BDNF have been reported, and any BDNF which is prepared by any method can be used in the present invention. When a BDNF isolated from animal tissues is used in the present invention, it may be purified to such a degree that it can be used as a medicament (cf., The EMBO Journal, vol. 5, p. 549 (1982)). Alternatively, BDNF can be obtained from a primary culture cell or an established cell line that can produce BDNF, and isolating from the culture broth thereof (e.g., culture supernatant, cultured cells). Moreover, a recombinant BDNF can be obtained by a conventional gene engineering technique, e.g., by inserting a gene encoding BDNF into a suitable vector, transforming a suitable host with the recombinant vector, and isolating from a culture supernatant of the resulting transformant (cf., Proc. Natl. Acad. Sci. USA, vol. 88, p. 961 (1991); Biochem. Biophys. Res. Commun., vol. 186, p. 1553 (1992)). The gene engineering technique is suitable for production of BDNF of same quality in a large scale. The host cells to be used in the above process is not limited, but may be any conventional host cells which have been used in a gene engineering technique, for example, *Escherichia coli, Bacillus subtilis,* yeasts, plant cells or animal cells.

The gene recombinant products of the other neurotrophins such as an NGF, an NT-3, an NT-4, etc. have been reported. NGF can be prepared from various host cells in the same manner as in the preparation of BDNF. (CHO cell, Schmelzer, C. H.: J. Neurochemistry, 59, 1675 (1992)), E. coli cell: Japanese Patent No. 2637392). NT-3 can be prepared from various host cells in the same manner as in the preparation of BDNF. The method for preparation thereof and assay thereof are disclosed in Neuron, vol. 4, 767 (1990), or JP-A-5-161493 (WO 91/3659). NT-4 can be prepared from various host cells in the same manner as in the preparation of BDNF. The methods for expression of the recombinant NT-4 and the methods for assay thereof are disclosed in Proc. Natl. Acad. Sci. USA, vol. 89, p. 3060 (1992), JP-A-4-509600 (WO 93/25684), or JP-A-6-501617 (WO 92/05254).

Among the insulin receptor agonists being used together with a neurotrophin, many medicaments containing mainly insulin are commercially available. Specific examples are animal-derived insulin "Iszilin" manufactured by SHIMIZU SEIYAKU-TAKEDA CHEMICAL INDUSTRIES, LTD.; recombinant human insulin "Novolin R" manufactured by Novo Nordisk-YAMANOUCHI PHARMACEUTICAL CO., LTD.; the same "Humulin R" manufactured by Eli Lilly Japan KK; and other crystalline insulin zinc suspension, isophane insulin suspension; insulin zinc protamine suspension, etc. (Drugs in Japan, edited by Japan Pharmaceutical Information Center, 1997 October-edition, p. 203-206). In addition, IGF-I is commercially available as "Somazon" from FUJISAWA PHARMACEUTICAL CO. LTD.

### PHARMACEUTICAL PREPARATION

A pharmaceutical preparation may be an injection preparation, an oral preparation, a liquid preparation, a lyophilized preparation, and the injection preparation for subcutaneous administration is preferable. These parenteral pharmaceutical preparations may contain a conventional stabilizer or a carrier used in this field, and it is preferable to use it in the form of an isotonic solution. The pharmaceutical carrier or diluent may be, for example, a protein derived from the serum such as albumin, an amino acid such as glycine, a saccharide such as mannitol, etc. When a pharmaceutical preparation of the present invention is in the form of a solution preparation or a lyophilized preparation, a surfactant such as Tween 80 is preferably added thereto in order to prevent aggregation of an active substance. Most briefly, a neurotrophin is dissolved in a commercially available insulin liquid preparation for subcutaneous administration to give a liquid preparation for subcutaneous administration of the present invention . When a prolonged pharmacological effect is required, then it is possible to prepare a pharmaceutical preparation with using a conventional microsphere-type sustained-release pharmaceutical carrier of biodegenerative polymer (e.g., polymer of polylactic acid-polyglycolic acid).

In the blood glucose level controlling agent of the present invention, the active ingredients, an insulin receptor agonist and a neurotrophin, may be administered simultaneously or separately, therefore, when administered simultaneously, these ingredients may be formulated into a preparation containing both ingredients, or these two ingredients may be formulated separately and set into a kit package. For example, these two ingredients are separately lyophilized, and a kit consisting of these two lyophilized ingredients and a solvent therefor is prepared, and then, these ingredients are dissolved when used, or a kit consisting of a solution preparation of one of these two ingredients and the other lyophilized ingredient is prepared, and then, these ingredients are mixed and dissolved when used, or a kit consisting of two solution preparations of each ingredient is prepared, and then, these two solution preparations are mixed when used.

When administered separately, these two ingredients are prepared in the form of the above kits, and each is administered separately, or a preparation of one of these two ingredient is used together with a commercially available preparation of the other ingredient.

### MIXING RATIO OF ACTIVE INGREDIENTS

The mixing ratio of the active ingredients, an insulin receptor agonist and an neurotrophin, can be freely defined and a suitable ratio can be determined by conventional animal experiments or clinical tests. In a subcutaneous injection preparation containing insulin and BDNF, BDNF is contained in an amount of 0.01 to 100 mg per 1 unit of insulin.

In the blood glucose level controlling agent of the present invention, an insulin receptor agonist and a neurotrophin are contained in an amount being suitable for a daily dose thereof, respectively. For example, they are formulated in a preparation containing an insulin receptor agonist in an amount of 0.1 to 1,000 units (converted amount of human insulin, hereinafter, the same) and a neurotrophin in an amount of 0.6 µg to 7,200 mg, preferably a preparation containing an insulin receptor agonist in an amount of 4 to 100 units and a neurotrophin in an amount of 6 µg to 3,000 mg. These active ingredients may be contained together in the same formulation as mentioned above, or contained in a separate preparation.

### USAGE

The blood glucose level controlling agent of the present invention can basically be administered to a patient to be insulinized, in a similar manner as the administration of a conventional insulin preparation. Basically, the daily dose of the insulin receptor agonist is in the range of 0.1 to 1,000 units/human, preferably 4 to 100 units/human, converted into an amount of human insulin. The daily dose of the neurotrophin which is administered simultaneously or separately with the insulin receptor agonist is in the range of about 0.01 µg to 120 mg/kg, preferably in the range of 0.1 µg to 50 mg/kg, and it can be administered subcutaneously, intravenously, or intramuscularly.

As mentioned above, in the blood glucose level controlling agent of the present invention, both of the active ingredients can be administered simultaneously or separately. Therefore, in the treatment of a diabetic patient to be insulinized by using the present blood glucose level controlling agent, the above daily doses of the insulin receptor agonist and the neurotrophin are administered to said patient simultaneously or separately. Preferably, the daily dose of the neurotrophin is administered once a day or divided into 2 to 3 dosage units, and then the daily dose of the insulin receptor agonist is administered once a day or divided into 2 to 3 dosage units.

### TOXICITY

In general, an insulin preparation is used by subcutaneous injection at a dose of 4 to 20 units before every meal at the early stage of diabetes, and then, according to the condition of the patient, it is used at a dose of 4 to 80 units/human/day. Although depending on circumstances, the dose up to about 100 units/human/day is confirmed to be clinically safe. When a neurotrophin, especially BDNF was administered subcutaneously to rats and cynomolgus monkeys at a dose of 100 mg/kg and 60 mg/kg, respectively, for four weeks, no animal was dead. With respect to the acute toxicity, BDNF was administered to rats and cynomolgus monkeys at a dose of more than 200 mg/kg, and no animal was dead. Therefore, BDNF shows high safety. With respect to other neurotrophins, no serious side effect has been reported either.

The present invention is illustrated by Examples.

### EXAMPLES

Effects of combined use of BDNF and insulin in diabetic animal model of which the secretion of insulin is reduced:
(1) Materials and method for experiment:
   Reagents: BDNF was purchased from REGENERON PHARMACEUTICALS, INC., and used. Commercially available saline solution (Otsuka Pharmaceutical Company, Limited), D-PBS (GIBCO), insulin (Novolin U injection 100 (trademark)), streptozotocin (Sigma) were purchased and used. The other reagents were commercially available ones with the best quality.
   Test animals: Male C57 BL/6NCrj mice (SPF) were purchased from Charles River Japan Inc. After one week pre-feeding, the animals were used in the experiment at 7 or 8 weeks old. The mice were kept in a room controlled at a temperature of 23±2°C under a humidity of 55±10 %, with an illumination cycle of light on (8:00 to 20:00) and light off (20:00 to 8:00). During the pre-feeding, the animals were freely fed with animal chaw (CE-2, Clea Japan, Inc.) and sterilized tap water, but if necessary, the mice were fasted when used in the experiment.
(2) Preparation o0f dosing solution of streptozotocin (STZ), BNDF, and insulin:
   STZ was dissolved in 10 mM sodium citrate solution (pH 5.5) to give a 20 mg/ml solution. The STZ solution was prepared when used, and used within 5 minutes after dissolution. A BDNF solution was prepared by diluting with D-PBS (Gibco) to a concentration of 2 mg/ml, and used in the experiment. An insulin solution was prepared by diluting a sustained release type of insulin (Novolin U injection 100) with saline and used in the experiment.
(3) Selection of and Grouping the hyperglycemic animals of which the secretion of insulin is reduced by STZ-administration:
   To the pre-fed mice was administered peritoneally the STZ solution prepared in the above (2) at a ratio of 10 µl/g (at a dose of STZ 200 mg/kg). On the following day, the STZ solution was administered again to the mice. Three days after the second administration of STZ, the blood glucose level and body weight of all of the mice were measured, and according to the results thereof, the animals were grouped. The mice showing no hyperglycemia (300 mg/dl or below), and the mice apparently being in bad conditions were excluded from the experiment.
(4) Method for measuring the blood glucose level and the statistic analysis thereof:
   The blood glucose level was measured in the blood taken from the tail vein by Antsence II blood glucose analyzer (Sankyo, Bayer). The blood glucose levels thus measured were analyzed by Turkey's test.
(5) Reduction in blood glucose level by administration of insulin alone:
   The relation between the insulin dose and the hypoglycemic activity thereof in the STZ-induced diabetic mice was studied. The hyperglycemic mice induced by STZ administration were grouped into 4 groups with respect to the blood glucose levels and the body weights thereof (6 animals per group). To each group was subcutaneously administered insulin at a dose of 0, 0.5, 1.5, and 5.0 units/kg, and the blood glucose level at every one hour after the administration was measured until 4 hours after the administration. After the administration of insulin, the animals were fasted. The blood glucose levels at each time in each group are indicated in Fig. 1. The dose-depending hypoglycemic effect by insulin administration was observed, and the significant reduction of the blood glucose level in the insulin 0.5 unit/kg-treated group (■) at one hour after the administration was observed as compared with that of the insulin 0 unit/kg-treated group (●), but the average blood glucose level of more than 400 mg/dl was merely reduced to about 300 mg/dl, and at two hours thereafter, the blood glucose level was almost recovered to the initial level. However, in the groups treated with insulin in a higher dose such as the 1.5 unit/kg-treated group (▲) and the 5.0 units/kg-treated group (○), the remarkable reduction of the blood glucose level was observed.
(6) Effect of combined use of BDNF and insulin (1); Effects on insulin of a low dose:
   The effect of a combined use of BDNF and insulin on the STZ-induced diabetic mice was studied. The mice having hyperglycemia induced by STZ-administration were grouped into 4 groups (9 or 10 animals/group) with respect to the blood glucose levels and the body weights thereof. The first group: D-PBS + saline-treated group (n=10), the second group: 20 mg/kg BDNF + saline-treated group (n=10), the third group: D-PBS + 0.5 unit/kg insulin-treated group (n=10), and the forth group: 20 mg/kg BDNF + 0.5 unit/kg insulin-treated group (n=9). BDNF (or D-PBS) was administered one hour prior to the insulin (or saline) administration, and at every one hour after the insulin (or saline) administration, the blood glucose level was periodically measured until 4 hours after the administration. The animals were fasted after the administration of BDNF or D-PBS. The blood glucose levels at each time in each group are indicated in Fig. 2.
   As a result, a significant decrease of blood glucose level was not observed in the 20 mg/kg BDNF + saline-treated group (□). On the other hand, the blood glucose level was reduced at 1 or 2 hours after the insulin administration to about 350 mg/dl in the D-PBS + 0.5 unit/kg insulin-treated group (▲). In the 20 mg/kg BDNF + 0.5 unit/kg insulin-treated group (○), the blood glucose level was remarkably reduced to about 210, 220 mg/dl at 1 or 2 hours after the insulin administration, respectively, and when compared with the result of the D-PBS + 0.5 unit/kg insulin-treated group, a significant reducing activity of blood glucose level was observed. That is, in the group treated with insulin of a low dose at which insulin does not exhibit a sufficient effect by administration alone, there was observed an apparent enhancement of insulin activity by pretreatment of BDNF.
(7) Effect of combined use of BDNF and insulin (2); effects on insulin of a higher dose:
   The effect of a combined use of BDNF and insulin of a higher dose on the STZ-induced diabetic mice was studied. The mice having hyperglycemia induced by STZ-administration were grouped into 4 groups (6 animals/group) with respect to the blood glucose levels and the body weights thereof. The first group: D-PBS + 1.5 unit/kg insulin-treated group, the second group: 20 mg/kg BDNF + 1.5 unit/kg insulin-treated group, the third group: D-PBS + 4.5 units/kg insulin-treated group, and the forth group: 20 mg/kg BDNF + 4.5 units/kg insulin-treated group. BDNF (or D-PBS) was administered one hour prior to the insulin (or saline) administration, and at every one hour after the insulin administration, the blood glucose level was periodically measured until 4 hours after the administration. The animals were fasted after the administration of BDNF or D-PBS. The blood glucose levels at each time in each group are indicated in Fig. 3. In Fig, 3, (a) shows the data when 1.5 unit/kg of insulin was used, and (b) shows the data when 4.5 units/kg of insulin was used.

As is shown in Fig. 3 (a) and (b), there was observed a remarkable reduction of the blood glucose level in each the D-PBS + 1.5 unit/kg insulin-treated group (△), the 20 mg/kg BDNF + 1.5 unit/kg insulin-treated group (■), the D-PBS + 4.5 units/kg insulin-treated group (○), and the 20 mg/kg BDNF + 4.5 units/kg insulin-treated group (◆), but there was no significant difference between the D-PBS-treated groups and the BDNF-treated groups.

### PREPARATION 1

Among the blood glucose level controlling agents of the present invention, a representative preparation, aqueous solution preparation containing insulin and BDNF for subcutaneous administration can be prepared as follows.
(1) To insulin (100 units) and purified recombinant BDNF (20 g) are added mannitol (10 mg) and non-ionic surfactant (polysorvate 80, 0.1 mg), and the mixture is dissolved in a 10 mM phosphate buffer (pH 7.0, 1 ml).
(2) BDNF (10 mg) is dissolved in an insulin injection preparation (Novolin U injection 100) of recombinant human insulin (1000 units/10 ml) manufactured by YAMANOUCHI Pharmaceutical Co., Ltd.

### PREPARATION 2

Among the auxiliary agents for insulin treatment of the present invention, a representative preparation, liquid injection preparation containing BDNF can be prepared by adding mannitol (10 mg) and non-ionic surfactant Polysolvate 80 (0.1 mg) to purified recombinant BDNF (20 mg), and dissolving the resultant in a 10 mM phosphate buffer (pH 7.0, 1 ml).

### INDUSTRIAL APPLICABILITY

By administering the blood glucose level controlling agent or the auxiliary agent of the present invention, (1) the insulin activity can be enhanced in a patient with hyperglycemia to be insulinized, but (2) the overaction of insulin is not enhanced so that the hypoglycemia can be prevented, by which the safe and efficient control of the blood glucose level of a diabetic patient within the normal range can be achieved. That is, as compared with agents containing insulin alone, the present invention can provide a method for treatment by which the blood glucose level can be controlled with the same degree as those of insulin of an original amount even though the dosage of insulin and the administration frequency thereof are reduced. Therefore, the present invention can achieve the improvement in the compliance, suppress the onset of hypoglycemia, and further ease the side effects such as macrovascular diseases in diabetic patients to be insulinized.

## Claims

1. A blood glucose level controlling agent for a diabetic patient to be insulinized, which comprises as the active ingredient an insulin receptor agonist and a neurotrophin.

2. The blood glucose level controlling agent according to claim 1, wherein the neurotrophin is a member selected from the group consisting of a brain-derived neurotrophic factor, a nerve growth factor, a neurotrophin 3, a neurotrophin 4, a neurotrophin 5, and a neurotrophin 6.

3. The blood glucose level controlling agent according to claim 1, wherein the neurotrophin is a brain-derived neurotrophic factor.

4. The blood glucose level controlling agent according to claim 1, wherein the insulin receptor agonist is an insulin.

5. The blood glucose level controlling agent according to claim 1, wherein the diabetic patient to be insulinized is a patient with insulin dependent diabetes mellitus.

6. The blood glucose level controlling agent according to any one of claims 1-4, which contains an insulin receptor agonist in an amount of 0.1 to 1000 units (converted amount of human insulin) and a neurotrophin in an amount of 0.6 µg to 7200 mg.

7. The blood glucose level controlling agent according to any one of claims 1-4, which contains an insulin receptor agonist in an amount of 4 to 100 units (converted amount of human insulin) and a neurotrophin in an amount of 6 µg to 3000 mg.

8. A blood glucose level controlling agent for a diabetic patient to be insulinized, which comprises as the active ingredient an insulin receptor agonist and a trkA, a trkB and/or a trkC receptor agonist.

9. An auxiliary agent for insulin treatment, which comprises as the active ingredient a neurotrophin.

10. A use of an insulin receptor agonist and a neurotrophin in preparation of a blood glucose level controlling agent for a diabetic patient to be insulinized.

11. The use according to claim 10, wherein the neurotrophin is a member selected from the group consisting of a brain-derived neurotrophic factor, a nerve growth factor, a neurotrophin 3, a neurotrophin 4, a neurotrophin 5 and a neurotrophin 6.

12. The use according to claim 10, wherein the neurotrophin is a brain-derived neurotrophic factor.

13. The use according to claim 10, wherein the insulin receptor agonist is an insulin.

14. A method for controlling the blood glucose level of a diabetic patient to be insulinized within the normal range, which comprises simultaneously or separately administering to said diabetic patient each effective amount of an insulin receptor agonist and a neurotrophin.

15. The method according to claim 14, wherein the insulin receptor agonist and the neurotrophin are administered respectively subcutaneously, intravenously or intramuscularly.

16. The method according to claim 14, wherein the neurotrophin is a member selected from the group consisting of a brain-derived neurotrophic factor, a nerve growth factor, a neurotrophin 3, a neurotrophin 4, a neurotrophin 5 and a neurotrophin 6.

17. The method according to claim 14, wherein the neurotrophin is a brain-derived neurotrophic factor.

18. The method according to claim 14, wherein the insulin receptor agonist is an insulin.
